# EUROPEAN PATENT APPLICATION

(11) **EP 3 545 834 A1**
(43) Date of publication of application: **02.10.2019**
(21) Application number: 18165315.5
(22) Date of filing: 30.03.2018
(51) Int. Cl.: A61B 5/0492, A61B 5/0408

(54) **ASSEMBLY FOR ATTACHING ELECTRODES TO A BODY**

(71) Applicant: Koninklijke Philips N.V., 5656 AE Eindhoven (NL)
(72) Inventor: DERKX, Rene Martinus Maria, 5656 AE Eindhoven (NL); DEVOT, Sandrine Magali Laure, 5656 AE Eindhoven (NL)
(74) Representative: de Haan, Poul Erik

(57) **Abstract**

The present invention relates to assembly for applying electrodes to a body. The assembly comprises a reference member to be disposed on an outer surface of a living body that has connection means for connection to a body. The assembly further comprises an electrode patch arranged to hold at least two electrodes in a pre-defined position. The reference member and the electrode patch are configured such that the electrode patch is rotatably held by the reference member, wherein the electrode patch can rotate with relation to the reference member.

## Description

### FIELD OF THE INVENTION

The field of the present invention relates to an assembly for attaching electrodes to a body. In particular the invention relates to electrodes that are applied to the skin of a subject for electromyography (EMG) measurements for the assessment of inspiratory respiratory-effort. The invention further relates to a method of applying electrodes to a body.

### BACKGROUND OF THE INVENTION

The placement of EMG electrodes is typically left to trained professionals. The placement of EMG electrodes can involve preparation of the skin where the EMG electrodes are to be placed. Such preparation can involve cleaning, shaving and abrasion of a topmost skin layer to remove dead skin cells. Nonetheless, significant intra-individual measurement variability can result from inconsistent electrode attachment. For most measurements involving at least two electrodes it is important that the electrodes are placed in the correct position and that the mutual distances between the electrodes is correct. For an untrained person this is quite a burdensome task.

It is known to use a patch carrying two electrodes having a fixed inter-electrode distance. This ensures that the mutual distances between the electrodes are fixed. However there are three degrees of freedom involved in the placement of such an electrode patch (horizontal offset, vertical offset and rotation of the patch), which makes it quite a challenge. Normally the electrodes carry an adhesive strip for attachment to the body. This means that after removal of the corresponding adhesive layer the patch must be placed in a single step to ensure accurate placement, while there is no possibility to allow for finetuning once one of the electrodes has been attached.

The assessment of inspiratory respiratory-effort by measuring the EMG-signals via two electrodes placed on the sternum is for example described in WO2013045920A1 and WO2016198288A1.

### SUMMARY OF THE INVENTION

It is an object of the present invention to facilitate the attachment of at least two electrodes to a living body. In particular, it would be advantageous to prevent incorrect placement of the electrodes such that even an untrained person can take up this task. Further, it is an object of the present invention to provide a method of applying electrodes to a body.

According to a first aspect of the present invention, an assembly for applying electrodes to a body is provided. The assembly comprises:
- a reference member to be disposed on an outer surface of a living body, the reference member having connection means for connection to a body;
- an electrode patch arranged to hold at least two electrodes in a pre-defined position, wherein the reference member and the electrode patch are configured such that the electrode patch is rotatably held by the reference member such that the electrode patch can rotate with relation to the reference member.

In a further aspect of the present invention a method for applying at least two electrodes to a body is provided. The method comprises the steps of:
- attaching a reference member on an outer surface of a living body at a reference position;
- providing an electrode patch arranged to hold at least two electrodes in a pre-defined position
- attaching the electrode patch to the reference member;
- rotating the electrode patch with relation to the reference member; and
- attaching the electrode patch to the body.

Preferred embodiments of the invention are defined in the dependent claims. It shall be understood that the claimed method can have similar and/or identical preferred embodiments as the claimed assembly and as defined in the dependent claims.

The present invention facilitates the placement of multiple electrodes, in particular multiple electrodes requiring a fixed inter-electrode distance.

In a first step the reference member is placed on a correct position of the body. In a second step the electrode patch holding at least two electrodes is connected to the reference member. The electrode patch, while being held by the reference member, is then rotated in the correct position. For example, while standing, a user can check in the mirror if the electrodes are at the same height. In a final step the electrode patch is fixed to the body. The attachment of the reference member could be further facilitated by having a medical professional make a temporary mark on a user's or patient's body indicating the correct position of the reference member. In comparison to the patch carrying two electrodes having a fixed inter-electrode distance as described in the background section, the present invention leaves only two degrees of freedom to be taken into account. By making the rotation independent from the two translational degrees of freedom, a user can divide placement of the electrode patch into two distinct sequential steps, which at least facilitates the (correct) placement of the patch.

Another advantage of the invention is that the reference-member can act as a strain-relieve in case the electrode patch carries extra weight or has cables attached to it. In this way, the two electrodes meant for the EMG measurement are less influenced by external forces on the patch which could otherwise compromise the quality of the EMG measurement.

The present invention is in particular useful for the assessment of inspiratory respiratory-effort. Herein one measures the electromyogram (EMG) signals via two EMG electrodes located at the second intercostal space symmetrically w.r.t. the sternum of a COPD patient. In this way, the activity of the muscles located close to the sternum (internal intercostal muscles) can be measured. A patient wears the two electrodes for a single or multiple days at the general ward of the hospital or the home. The amount of respiratory-effort can be derived from the voltage measured across the two EMG electrodes (differential measurement) during the inhalation-phase. The amount of respiratory-effort is an important vital sign, e.g. for the detection of exacerbation for COPD patients in the hospital or the home. Next to the respiratory-effort measured via the EMG electrodes, the patch can also include other sensor modalities which require accurate placement on the chest, for example reflective SpO2 sensors that measures the oxygen levels in the blood through the vessels, e.g. also located close to the sternum.

In an embodiment the assembly further comprises at least one electrode that is attachable to the electrode patch member, and wherein the at least one electrode has an adhesive portion. Preferably the adhesive portion is covered by a protective removable layer, which further facilitates the connection to a body. Alternatively, the electrode patch comprises connection means for connection to a body for facilitating the attachment of the patch to a body.

In another embodiment the reference member comprises an electrical connection means for electrical connection to a body. The reference member can then act as a grounding electrode. Using a reference-electrode can help for better powerline noise reduction. The quality of the differential measurement via the two electrodes is affected by power line interference. This power line interference can generate a so-called common mode voltage on the patient, but due to the limited 'common mode rejection ratio' (CMRR) of the differential amplifier, it can still lead to power line interference on the output of the differential amplifier. Also an inbalance in the skin-electrode impedance and the wiring can lead to more power line interference on the output of the differential amplifier. To further reduce this powerline interference, one can use a grounding electrode. This grounding electrode is connected to the output of an amplifier that uses the measured common mode voltage and drives (via a feed-back loop) a small current via this third grounding electrode to counteract the common mode voltage on the patient. Accordingly this embodiment the total CMRR of the differential amplifier is enhanced.

In yet another embodiment the electrode patch comprises at least one extension, the at least one extension being arranged to hold at least one electrode, wherein the extension is connected to a base part of the electrode patch by means of a flexible element. This enables easy attachment of the electrode patch to a body, in particular when the base part is used to attach the electrode patch to the reference member.

For an easy coupling and facilitating the rotational movement the reference member preferably comprises a dome-shaped element and the electrode patch comprises a dome-shaped counter-element. The dome-shaped element can be inserted into the dome-shaped counter-element or vice versa. Preferably the dome-shaped element and the dome-shaped counter-element are mutually connectable via a snap-fit connection. A further preference is using springs to fix both elements. For example, one can apply 2 blade springs that engage the dome-shaped elements from opposite sides when making the connection.

Preferably the electrode patch is arranged to hold other sensor modalities, such as an optical-sensor to measure blood pulses or an SpO2 sensor to measure ogygen levels of the blood. This is in particular advantageous in case of inspiratory respiratory-effort measurement measured parasternally, i.e. close to the sternum, in an intercostal space, while a lot of vital arteries can be assessed through the parasternal intercostal space. For example blood oxygenation can be measured by optical techniques, such as photoplethysmography (PPG), because oxygenated and deoxygenated hemoglobin have a different color. This can be exploited by transmitting light having two different optical wavelengths (typically at red and infrared colors) through tissue (usually one measures at the finger-tip, but one can also measure at the parasternal intercostal space) and then measuring the light intensity with a photodiode (usually this is done in a transmissive mode via the other side on the finger-tip, but it can also be done in a reflective mode at the parasternal intercostal space). Such a method for measuring arterial oxygenation with PPG is called "pulse oximetry", and the arterial oxygenation measured in this way is referred to as "SpO2". An SpO2 sensor is arranged to derive the SpO2 value, by comparing the relative changes in detected light (e.g. changes that are assumed to be caused by arterial blood because they pulsate due to the heart beat, as compared to the slower fluctuating component) at one wavelength (λ1) to those at another wavelength (λ2), with other absorption characteristics for oxygenated and deoxygenated hemoglobin, resulting in the following ratio of ratios (RR): RR=(AC/DC)_{λ1}/(AC/DC)_{λ2}. This ratio is subsequently used in a formula to derive the arterial oxygenation. Often, a linear relationship is assumed: SpO2=a·RR+b, wherein a and b are calibration constants.

To further facilitate proper connection of the assembly to the body, the electrode patch in a preferred embodiment comprises a visual marking, preferably a straight line. By means of such a line a user can check that a correct rotation of the patch has been performed, for example when the visible line has a substantially vertical orientation with relation to a ground surface.

To even further facilitate proper connection of the assembly to a body the electrode patch in a preferred embodiment comprises means that indicate that an imaginary center-line through two electrodes being disposed on the patch runs substantially orthogonal to the gravity axis. The most obvious example would be to use a plumb rule. In this way a user can easily check, e.g. by looking in a mirror while standing up, if two electrodes provided at the electrode patch are at the same height level and thus at the correct position.

### BRIEF DESCRIPTION OF THE DRAWINGS

These and other aspects of the invention will be apparent from and elucidated with reference to the embodiment(s) described hereinafter. In the following drawings
Fig. 1A shows an assembly for applying electrodes to a body in dis-assembled state;
Fig. 1B shows an assembly for applying electrodes to a body in assembled state;
Fig. 2A shows a top-view and a side-view of a central part of the electrode patch, the side view taken from the line A-A as indicated in the top-view;
Fig. 2B shows a top-view and a side-view of the reference member, the side view taken from the line A-A as indicated in the top-view;
Fig. 2C shows a side view of the reference member and the electrode patch in an assembled state;
Fig. 3 shows the locations of the electrodes across the sternum in case of inspiratory respiratory-effort measurements.

### DETAILED DESCRIPTION OF THE INVENTION

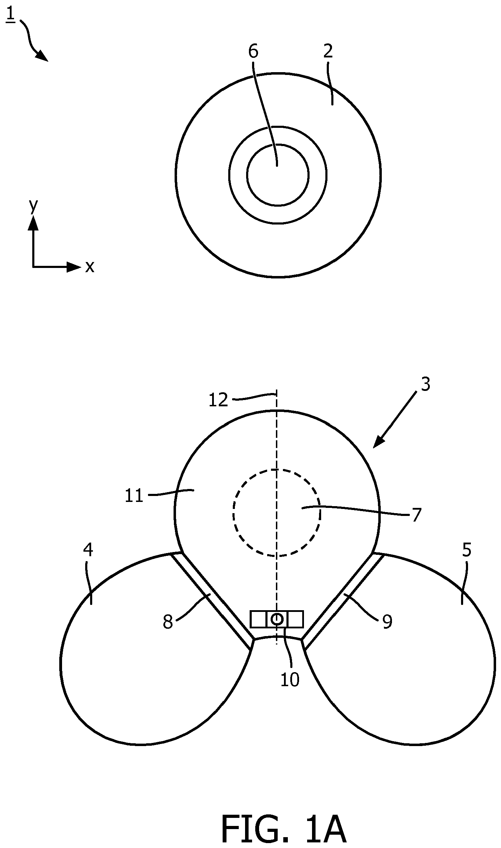

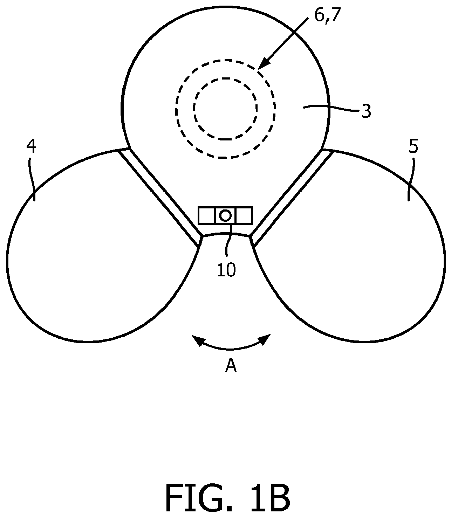

Figs. 1A and 1B both show an embodiment of the an assembly 1 for applying electrodes to a body. The assembly comprises a reference member 2 to be attached on an outer surface of a living body. The reference member 2 has connection means for connection to a body (not shown) such as an adhesive strip, provided at an underside.

The assembly 1 further comprises an electrode patch 3 arranged to hold at least two electrodes in a pre-defined position. In the embodiment shown two electrodes 4,5 are pre-disposed and integrated. However it can also be that the electrodes are connected to the assembly only after the assembly is disposed on an outer surface of a living body (via the reference member).

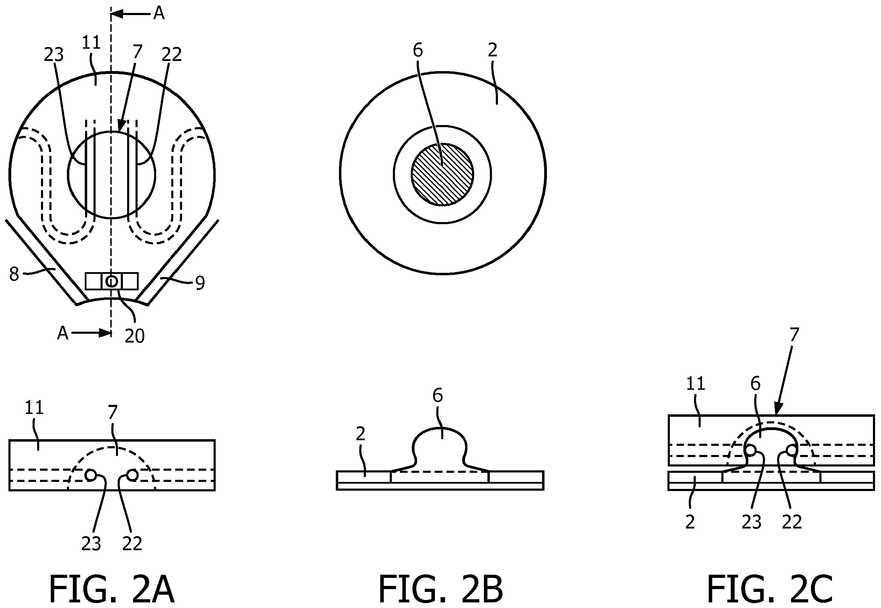

For ease of connection the reference member 2 has a dome-shaped element 6 and the electrode patch 3 has a dome-shaped counter element 7 (see also Figures 2A-2C). Upon connection the dome-shaped element 6 is inserted in the dome-shaped counter element 7.

Preferably the connection between the reference member 2 and the electrode patch 3 comprises a snap-fit connection as shown in Figs. 2A-2C. In the assembled state the electrode patch 3 is held by the reference member 2 but can still rotate with relation to the reference member.

The electrode patch 3 may comprise a plumb rule 10 to indicate that the electrodes are at the same height with respect to a (flat) ground surface. If a user stands upright this automatically means that the electrodes are at the correct position or at least the correct position with relation to the reference member.

The electrodes 4,5 are preferably arranged on an extension relative to a central part 11 of the electrode patch 3. The extensions are connected to the central part by means of flexible elements 8 and 9 that enable to flip the electrodes 4,5 with relation to the central part 11 of the electrode patch. In this way, the two electrodes 4 and 5 are easily accessible by the user when the reference member has been attached to the body and the patch has been assembled on the reference member. In the figures each electrode is attached to an individual extension. However it is also possible to attach two or a plurality of electrodes on a single extension that is connected to the central part by a (single) flexible element.

In case the electrodes 4 and 5 are integrated in the patch, the user can easily remove the adhesive protection layer covering the electrodes 4 and 5 and attach the electrodes to the body. In case of separate electrodes 4 and 5, the user can first connect the electrodes to the extensions, remove the protective layer of the two connected electrodes 4 and 5 and attach the two electrodes with an adhesive layer or strip on a body.

When applying the assembly 1 in a first step one places the reference member 2 on the sternum or on another preferred location on a body. The reference member 2 maybe a single disposable electrode which has an adhesive part and is placed separately from the electrode patch. The attachment of the reference member 2 may be guided by a single (semi-permanent) marker on the sternum or other location of the body made by a medical professional. Alternatively, the medical professional can indicate to the user on which anatomical landmark the reference member 2 has to be placed. For example, on the sternum at the level of the first intercostal space. By palpation with two fingers, the user can find the borders of the sternum and attach the reference member 2 in-between these borders. In a next step one places the electrode patch 3 on the reference member 2 by inserting the dome-shaped element 6 into the dome-shaped counter element 7. A snap-fit connection makes that the electrode patch 3 is held by the reference member 2. Next the electrode patch 3 is rotated in the correct position which may be obtained by checking the air bubble of the plumb rule. Alternatively, the user can look at a center-line that is printed on the electrode patch 3 as shown in Fig 1A. In Fig 1B such rotation is indicated by arrow A. Finally one removes a protective layer of both electrodes or at least one electrode and makes the connection to a body by means of an adhesive strip covered by the layer (not shown). This greatly facilitates a correct positioning while the rotation is made independent from the correct translation position (X,Y position).

Fig. 2A shows a top-view and a side-view of a central part 11 of the electrode patch. Next to the elements known from Figs 1A this figure shows two springs 22, 23 that are located in an internal space of the central part 11. The top view is the upper figure and the side view is the lower figure.

Fig. 2B shows a top-view and a side-view of the reference member 2. The side view from the lower figure illustrates the shape of the dome-shaped element 6 in more detail.

Fig. 2C shows a side view of the reference member 2 and the electrode patch in an assembled state. The dome-shaped element 6 first is inserted into the dome-shaped counter-element 7 while pushing the springs 22, 23 outside. Once the widest part of the dome-shaped element has passed the springs 22, 23 fixate the dome-shaped element 6. As a result the electrode patch is hold by the reference member 2 such that the electrode patch can still rotate with relation to the reference member.

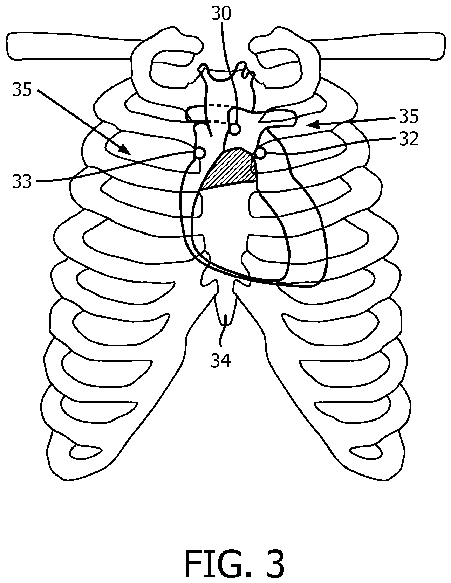

Fig.3 shows the locations of the reference member 30 and both measuring electrodes 32, 33 across the sternum 34 in case of inspiratory respiratory-effort measurements. Both electromyogram (EMG) electrodes measure the respiratory muscle activity at the second intercostal space 35. It is essential that both electrodes are placed close to the sternum in order to have a correct measurement.

Optionally a grounding-electrode is used by using the reference member as the grounding-electrode. Using a reference-electrode can help for better powerline noise reduction, while the quality of the differential measurement via the two electrodes is badly affected by power line interference.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage. It should be noted that optionally more than two electrodes can be provided for the EMG measurement. An electrode can optionally comprise more than two electrically insulated electrode areas.

Any reference signs in the claims should not be construed as limiting the scope.

## Claims

1. Assembly (1) for applying electrodes to a body comprising:
- a reference member (2) to be disposed on an outer surface of a living body, the reference member having connection means for connection to a body;
- an electrode patch (3) arranged to hold at least two electrodes (4,5) in a pre-defined position, wherein the reference member and the electrode patch are configured such that the electrode patch is rotatably held by the reference member, wherein the electrode patch can rotate with relation to the reference member.

2. Assembly (1) according to claim 1, wherein the reference member (2) comprises an electrical connection means for electrical connection to a body.

3. Assembly (1) according to any one of claims 1 or 2, wherein the assembly further comprises at least one electrode that is attachable to the electrode patch, and wherein the at least one electrode has an adhesive portion, the adhesive portion preferably being covered by a protective removable layer.

4. Assembly (1) according to any one of claims 1-3, wherein the electrode patch (3) comprises at least one extension, the at least one extension being arranged to hold at least one electrode (4,5), wherein the extension is connected to a base part of the electrode patch by means of a flexible element (8,9).

5. Assembly (1) according to any one of claims 1-4, wherein the reference member (2) comprises a dome-shaped element (6); and wherein the electrode patch (3) comprises a dome-shaped counter-element (7) such that the dome-shaped element can be inserted into the dome-shaped counter-element or vice versa.

6. Assembly (1) according to any one of claims 1-5, wherein the dome-shaped element (6) and the dome-shaped counter-element (7) are connectable via a snap-fit connection.

7. Assembly (1) according to any one of claims 1-6, wherein the electrode patch (3) is arranged to hold other sensor modalities, preferably an optical sensor.

8. Assembly (1) according to claim 7, wherein the sensor modality is an SpO2-sensor.

9. Assembly (1) according to any one of claims 1-8, wherein the electrode patch (3) comprises a visual marking (12) for facilitating correct positioning, preferably a straight line.

10. Assembly (1) according to any one of claims 1-9, wherein the electrode patch (3) comprises means (10) to indicate that an imaginary center-line running through two electrodes being disposed on the patch and the gravity axis are substantially orthogonal with relation to each other.

11. Method for applying at least two electrodes to a body comprising the steps of:
- attaching a reference member (2) on an outer surface of a living body at a reference position;
- providing an electrode patch arranged (3) to hold at least two electrodes in a pre-defined position
- attaching the electrode patch to the reference member such that the electrode patch is held by the reference member but can rotate with relation to the reference member;
- rotating the electrode patch with relation to the reference member to a required position; and
- attaching the electrode patch to the body.

12. Method according to claim 11, wherein the method further comprises the step of;
- providing two or more electrodes (4,5), the electrodes having connection means;
- attaching the electrodes to the electrode patch;
- attaching the electrode patch to the body via the connection means.

13. Use of an assembly (1) according to any one of claims 1-10 for the measurement of inspiratory respiratory-effort.

14. Electrode patch (3) for use in an assembly (1) according to any one of claims 1-10.
